Europäisches Patentamt

European Patent Office

Office européen des brevets

(11) Publication number: **0 280 196**
**A2**

# EUROPEAN PATENT APPLICATION

(21) Application number: 88102379.0

(22) Date of filing: 18.02.88

(51) Int. Cl.⁴: **H04Q 7/02** , H04Q 9/00 , A61B 5/00

(30) Priority: 23.02.87 JP 39944/87

(43) Date of publication of application:
31.08.88 Bulletin 88/35

(84) Designated Contracting States:
DE ES FR GB IT NL SE

(71) Applicant: **Nihon Kohden Corporation**
**31-4, 1-chome, Nishiochiai**
**Shinjuku-ku Tokyo(JP)**

(72) Inventor: **Inahara, Kazuo**
**No. 16-1, Wakitashinmachi**
**Kawagoe-shi Saitama-ken(JP)**
Inventor: **Igarashi, Junichi**
**No. 10-11, Higashimagome 1-chome**
**Ohta-ku Tokyo(JP)**

(74) Representative: **MEISSNER, BOLTE &**
**PARTNER**
**Widenmayerstrasse 48 Postfach 860624**
**D-8000 München 86(DE)**

(54) Apparatus for managing channels of a radio communication system.

(57) A channel management apparatus provided for a plurality of master radio communication units ($1_1$..... $1_n$, $2_1$..... $2_n$) which perform communication by selecting as desired one or more of a plurality of channels (1ch..... nch) that are respectively assigned to a plurality of radio communication slave units. This apparatus is provided with a shared storage device (3) which is capable of being set to each master unit ($2_1$......$2_n$) and which contains items of data on registered channels (1ch..... nch) which correspond to all of the channels used by the master units, and a plurality of groups of devices incorporated in the master units. Each of the groups of devices includes: a setting device (5) for manually setting the channels to be used by a corresponding one of the master units; a display device (6) for displaying the channels used by the master units; a channel data processing device (7) adapted to make the display device (6) display data on the register channel by reading the data from the storage device (3) which has been set, the data processing device (7) updating the data on the registered channels in the storage device (3) in accordance with the data on the channels used by the master unit supplied from the setting means (5); and a channel selecting device (8) adapted to select, in accordance with the data on the channels to be used, channels through which the communication is performed.

FIG.1

# Apparatus for managing channels of a radio communication system

## Background of the invention

This invention relates to an apparatus which controlls and manages the channels of a radio communication system in a limited region or an institution, e. g. a hospital, which enables each of a plurality of master units of the radio communication system to perform communication with a plurality of slave units by selecting desired ones of the channels specifically assigned to those slave units.

If radio communication is performed by a plurality of slave units and a plurality of master units in charge of respective shares of operation, the management of channels is very important to avoid double or simultaneaous use of one and the same channel at a given time. In a conventional method of channel management, determination which channels are in use and which are free is carried out through a separate medium by a man or a computer, and this operation is not linked with master units. As a result, even if channels selected to be used have been correctly registered, there is a strong possibility of inhibited channels being used, causing problems such as radio interference.

## Summary of the invention

Therefore, it is an object of the present invention to provide a channel management apparatus which improves the reliability of the channel management in a radio communication system in which uni-direction or bidirectional communication is performed between a plurality of radio communication slave units to which channels are assigned and a plurality of radio communication master units which select the channels as desired.

To this end, the present invention provides a channel management apparatus provided for a plurality of radio communication master units which perform communication by selecting as desired one or more of a plurality of channels that are respectively assigned to a plurality of radio communication slave units, the apparatus having a shared storage device which is capable of being set to each master unit and which contains items of data on registered channels which correspond to all of the channels used by the master units, and a plurality of groups of devices incorporated in the master units. Each of the groups of devices includes: a setting device for manually setting the channles used by a corresponding one of the master units; a display device for displaying the channels used by the master unit as usable or unusable channels; a channel data processing device adapted to make the display device display data on the registered channels by reading this data from the storage device which has been set, the data processing device updating the data on the registered channels in the storage device; and a channel selecting device adapted to select, in accordance with the data on the channels to be used, channels through which the communication is performed.

In accordance with the present invention, when radio communication is performed by a plurality of master units in charge of respective shares because there are a plurality of slave units, the provision of a shared storage device facilitates the management of channel assignment to the master units, thereby preventing problems such as radio interference.

The above and other objects, features, and advantages of the present invention will become readily apparent form the following description when taken in conjunction with the accompanying drawings in which a preferred embodiment of the present invention is shown by way of illustrative examples.

## Brief description of the drawings

Fig. 1 is a circuit block diagram of a channel management apparatus which has a constitution according to the principle of the present invention;

Fig. 2 is a perspective view of a radio communication unit in accordance with an embodiment of the present invention;

Fig. 3 is a circuit diagram of the embodiment relating to Fig. 2; and

Fig. 4 is a flow chart of the operation in accordance with the embodiment of the present invention relating to Fig. 2.

## Description of a preferred embodiment

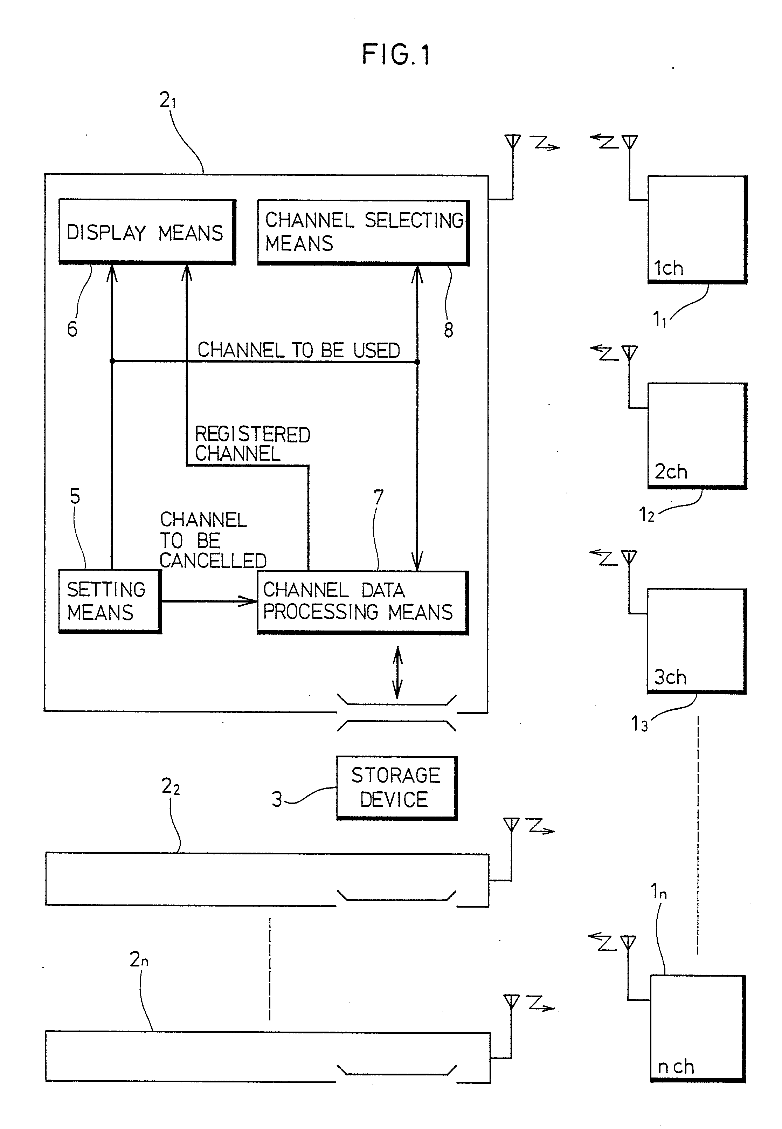

Referring to Fig. 1, a channel management apparatus is shown which is constructed as described below and which comprises a plurality of radio communication master units $2_1$ to $2_m$ which perform communication by selecting one or more of a plurality of channels 1ch to nch that are respectively assigned to a plurality of radio communication slave units $1_1$ to $1_n$.

A shared storage device 3 is provided which is capable of being set to any of the desired master units $2_1$ to $2_m$ and which is constituted by an EEPROM, floppy disk, etc.. Such an EEPROM contains items of data on registered channels which correspond to channels used by the master units $2_1$ to $2_m$. Each of the master units $2_1$ to $2_m$ comprises the following components or units: setting means 5 for manually setting channels which are used by that master unit and channels which are to be cancelled; display means 6 adapted to display information about the channels, for example, usable channels and the set channels used by this master unit, wherein the display means 6 may be constituted by a cathode ray tube, a numeric value indicator, recording paper, etc.; data processing means 7 which makes the display means 6 display data as to registered channels as usable or unusable channels by reading these data from storage device 3 which has been set, and which updates the data as to registered channels in the storage device 3 in accordance with set data on the channels used by this master unit, and similarly updates the data on registered channels in accordance with the data on channels to be cancelled; and channel selecting means 8 which operates to select, in response to the setting of channels which are to be used, channels through which the communication is performed.

The above system operates as described below.

If the master unit $2_1$ is to be operated, the storage device 3 is set to this master unit $2_1$. The channel data processing means 7 reads out data stored in the storage device 3 on registered channels relating to other operating master units, and makes the display means 6 display the usable and unusable channels, respectively.

That is, if channels which are in use have been stored as registered data, those channels are directly displayed as unusable channels or, after calculation, they are displayed as unusable channels. If channels which are in use have been stored as registered data on usable channels after preliminary calculation, they are directly displayed as usable channels. If a channel which the respective master unit desires to use is set by the setting means 5 on the basis of the displayed channels, it is displayed on the display means 6 for confirmation. In accordance with this setting, the channel selecting means 8 enables this master unit to communicate with the slave unit $1_1$ of the corresponding channel, and, at the same time, the channel data processing means 7 updates the registered data in the storage device 3 by erasing the data on usable channels or writing the data on unusable channels in accordance with the data on the channels used by this master unit.

If a registered channel is to be cancelled, the channel to be cancelled which will not be used is set by the setting means 5, so that the channel selecting means 8 is stopped from selecting that channel while the channel data processing means 7 correspondingly updates the data in the storage device 3.

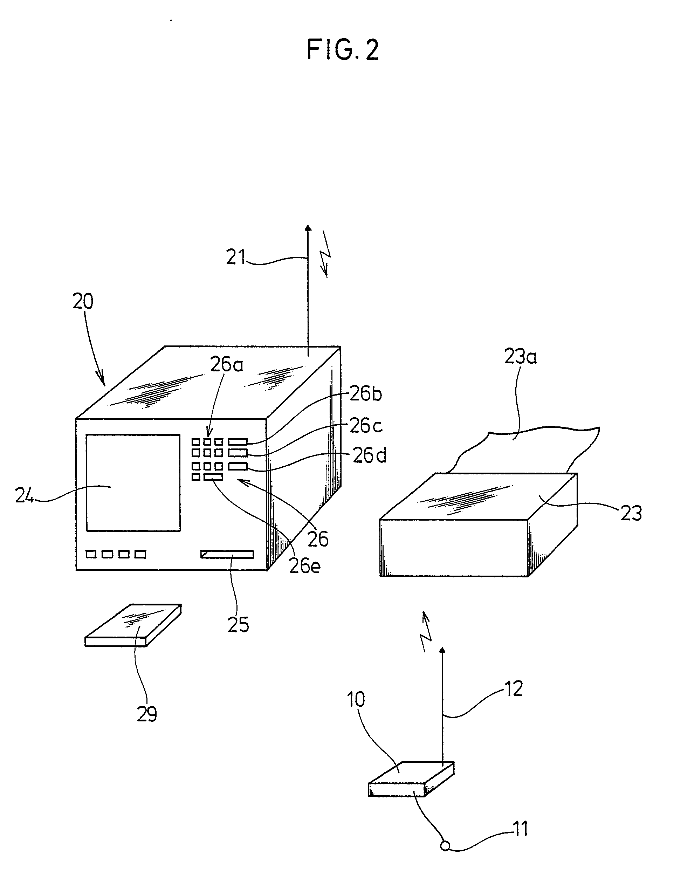

Figs. 2 to 3 show an embodiment in which the present invention is applied to an electrocardiogram telemeter used in a hospital.

Referring to Fig. 2, 150 slave units 10, for example, are provided in the hospital. Each slave unit 10 is adapted to transmit an electrocardiogram signal induced through an electrode 11. Several dozen master units 20 are provided in the hospital. In each master unit 20, a receiving section selectively receives through an antenna 21 signals which are transmitted from slave units 10 through 150 channels assigned to the slave units 10. A recorder 23 is attached to each master unit 20.

The panel of each master unit 20 is provided with a display, e.g. a cathode ray tube 24, a slot 25 into which a storage device 29 in the form of a card incorporating a floppy disk is removably set, and a keyboard 26 which is provided as a setting means and which is constituted by a ten-key key group 26a for setting channels, a registration key 26b, a cancellation key 26c, a confirmation key 26d, and an end key 26e. The recorder 23 and the cathode ray tube 24 are capable of recording and displaying, respectively, electrocardiogram waveforms supplied through four channels.

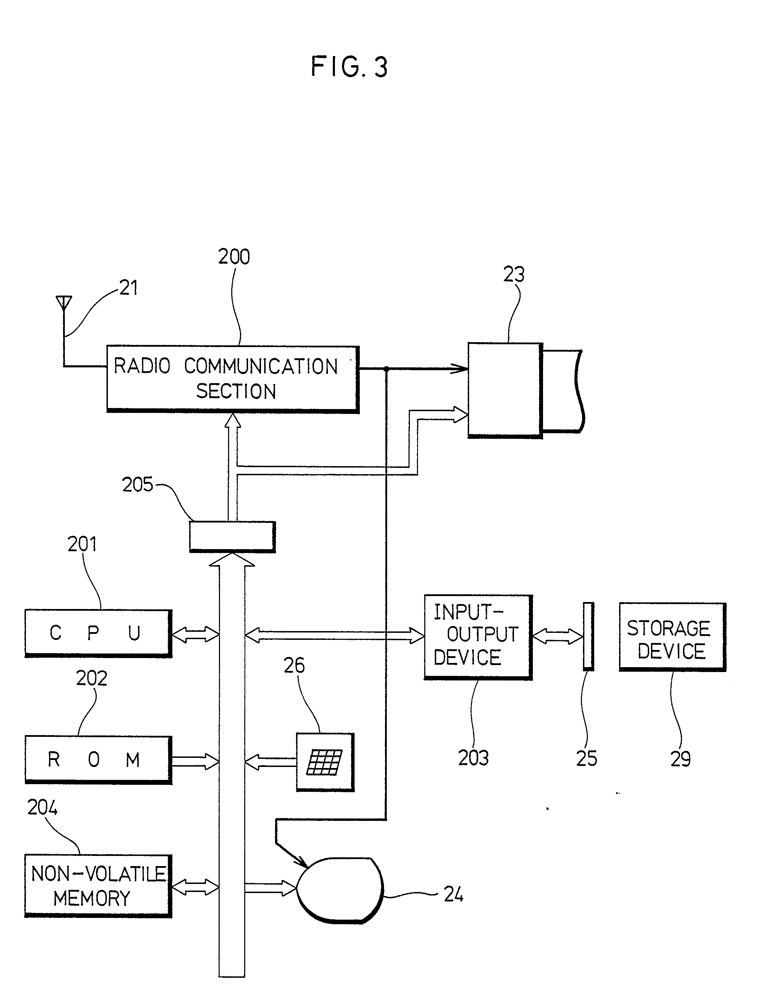

Fig. 3 shows the circuit arrangement of the master unit 20. From the antenna 21, a radio communication section 200 receives signals, for example electrocardiogram signals, on four channels selected by a channel change-over circuit incorporated in a receiving section, and outputs detected waveform signals obtained therefrom to the recorder 23 and the cathode ray tube 24.

A CPU 201 is adapted to perform data processing of data supplied from the storage device 29 and the keyboard 26 in accordance with a channel management program and stationary data stored in a ROM 202, as described hereinafter. An input-output device 203, including a floppy disk deck provided with the slot 25 is adapted to carry out data exchange between the CPU 201 and the storage device 29. A non-volatile memory 204 maintains data on channels used by this master unit and the card number of the storage device 29, even after the power for the unit has been cut. As shown in Fig. 3, the radio communication system 200 and the respective data processing units are connected by respective buses and through an interface unit 205.

The CPU 201 operates as decribed below. First, the CPU 201 reads data on the card number from the storage device 29, compares these data with the corresponding data stored in the non-volatile memory 204, and sends, in the case of non-coincidence, non-coincidence data to the cathode ray tube 24 to display a registration-unable state. If the card number has not been registered, the CPU 201 stores it in the non-volatile memory 204.

After the registration key 26b has been set, the CPU 201 receives these key data, reads the data on registered channels stored in the storage device 29, calculates usable channels from the data on all channels previously written in the ROM 202, and displays them on a recording medium 23a, e.g. on recording paper of the recorder 23.

When four channels to be used are set by the ten-key key group 26a so that an item of key data is produced each time the end key 26e is operated, the CPU 201 successively sends items of data on the channels to be used to the cathode ray tube 24 in response to the items of key data.

Thereafter, the CPU 201 determines, in response to key data produced by the operation of confirmation key 26d, whether or not these channels overlap the registered channels, and displays an OK state when the check is positive or an NG state, when the check is negative on the cathode ray tube 24. When the end key 26e is operated in the OK state, the CPU 201 activates, in response to this, the channel-change-over circuit incorporated in the radio communication section 200 so as to enable reception through the set four channels, and it registers through the input-output device 203 the data on channels to be used.

If the cancellation key 26c is operated and if the data on registered channels have not been displayed, the CPU 201 displays, on the cathode ray tube 24, these data, the data on the channels to be used read out from the non-volatile memory 204, and the data on a channel to be cancelled which has been set by the ten-key key group 26a and the end key 26e.

Then, in response to the operation of the confirmation key 26d, the CPU 201 determines whether or not the channel to be cancelled is included in the channels to be used, thereafter displaying an OK state or an NG state. When the end key 26e is operated in response to OK, the CPU 201 erases the data on the corresponding registered channel in the storage device 29 and the content of the non-volatile memory 204 and cancels the reception of the channel to be cancelled.

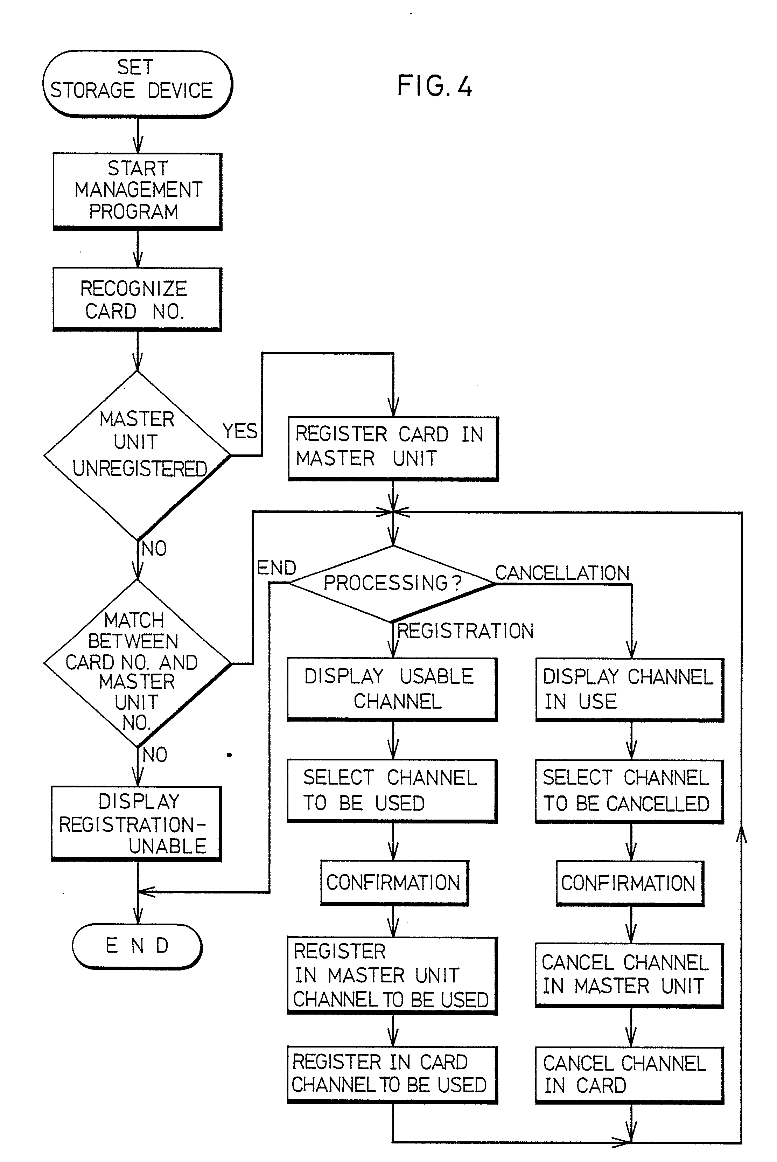

The operation of the thus-constructed management apparatus will be described below with reference to the flow chart of Fig. 4.

If one of the master units 20 is to be newly operated, the storage device 29 is set into the slot 25 of that master unit 20. The CPU 201 starts the management program, compares the card number with the registered card number, and displays on the cathode ray tube 24 the registration-enable state in case of coincidence or the registration-disable state in the case of non-coincidence.

If the card number has not been registered, the registration-enable state is displayed after the registration of the card number. When the operator operates the registration key 26b, the data on registered channels stored in the storage device 29 are displayed on the display 24 and recorded on the recording paper 23a. Of the usable channels displayed on the display and recorded on the recording paper 23a, at most four channels which are used by this master unit 20 are successively input through the ten-key key group 26a and are displayed by the operation of the end key 26e.

After the OK display has been effected by operating the confirmation key 26d, the end key 26e is operated, thereby making the radio communication section 200 select these channels and while performing writing into the non-volatile memory 204 and the storage device 29.

To cancel the use of a channel which has already been registered, the cancellation key 26c is operated so as to display the channels which are in use, and the channel to be cancelled is also displayed by operating the end key 26e. The confirmation key 26d is operated so as to confirm that this channel is not a channel included in the registered channels for other master units 20 but a channel used by this master unit. If the OK display is effected, the end key 26e is operated, thereby interrupting the selecting operation of the radio communication section 200 and cancelling registration in the storage device 29 and the non-volatile memory 204.

In accordance with the above-described embodiment, the unit number of each master unit 20 may be stored in the ROM 202 and stored in the storage device 29 together with the data on the channels which are in use, thereby enabling indication of which master unit is using which channel.

This method is particularly suitable for a master unit 20 without a non-volatile memory 204 in order to know the channels which this unit is allowed to use, when this unit is started again after cutting off the power supply. The present invention can also be applied to a radio communication system in which the master unit 20 does not only display an electrocardiogram waveform but also incorporates an analyzer for analyzing the waveform or controls the slave unit 10, or in which the slave unit 10 does not only select its discriminated channel but also a plurality of channels through which a plurality of kinds of organism signals are transmitted in linked relationship with the discriminated channel.

If the management apparatus is adapted to make a plurality of master units 20 receive signals from one slave unit 10, it is necessary to change the above-described program of the operation of confirmation. It is also possible to display the unit number of a master unit by assigning a channel which is used by this master unit.

## Claims

1. A channel management apparatus in a radio communication system provided for a plurality of radio communication master units (20) which perform communication by selecting as desired one or more of a plurality of channels that are respectively assigned to a plurality of radio communication slave units (10), the apparatus comprising:
- a shared storage device (3; 29) capable of being set to each master unit ($2_1$..... $2_n$; 20) and adapted to contain data on registered channels (1ch.... nch) which correspond to all of the channels used by the master units;
- and a pluraltiy of groups of means incorporated in the master units ($2_1$.....$2_n$; 20) wherein each of the groups of means includes:
- setting means (5; 26) for manually setting channels used by a corresponding one of the master units ($2_1$...... $2_n$; 20) and for cancelling channels to be cancelled;
- display means (6; 23, 24) for displaying usable or unusable channels and the channels used by the master unit ($2_1$.....$2_n$; 20);
- data processing means (7; 201 - 205) adapted to make the display means (6; 23, 24) display data on the registered channels as usable or unusable channels by reading the data on the registered channels from the storage device (3, 20) which has been set, the data processing means (7; 201 - 205) updating the data on the registered channels in the storage device (3; 29) in accordance with the data on the channels used for the master unit (20) and the data on the channel to be cancelled supplied from the setting means (3; 26); and
- channel selecting means (8) adapted to select, in accordance with the data on the channels to be used, channels through which the communication is performed.

2. The channel management apparatus according to claim 1, wherein each of the groups of means includes a non-volatile memory (204) for storing data on channels used by the corresponding one of the master units ($2_1$...... $2_n$; 20).

3. The channel management apparatus according to claim 1 or 2, wherein the channel data processing means (7; 201 - 205) comprises a CPU (201).

4. The channel management apparatus according to any of claims 1 to 3, wherein the storage device (3; 29) is a card type of storage device.

# FIG.1

# FIG. 2

# FIG. 3

# FIG. 4

SET STORAGE DEVICE

START MANAGEMENT PROGRAM

RECOGNIZE CARD NO.

MASTER UNIT UNREGISTERED

YES → REGISTER CARD IN MASTER UNIT

NO

MATCH BETWEEN CARD NO. AND MASTER UNIT NO.

NO → DISPLAY REGISTRATION-UNABLE

END

PROCESSING ?

END

CANCELLATION

REGISTRATION

DISPLAY USABLE CHANNEL

SELECT CHANNEL TO BE USED

CONFIRMATION

REGISTER IN MASTER UNIT CHANNEL TO BE USED

REGISTER IN CARD CHANNEL TO BE USED

DISPLAY CHANNEL IN USE

SELECT CHANNEL TO BE CANCELLED

CONFIRMATION

CANCEL CHANNEL IN MASTER UNIT

CANCEL CHANNEL IN CARD